# EUROPEAN PATENT APPLICATION

(11) **EP 2 216 025 A2**
(43) Date of publication of application: **11.08.2010**
(21) Application number: 10163570.4
(22) Date of filing: 07.02.2003
(51) Int. Cl.: A61K 31/55, A61P 1/00

(54) **Use of acylaminoalkenylene-amide derivatives in irritable bowel syndrome and functional dyspepsia**

(30) Priority: 08.02.2002 GB 0203061
(62) Divisional of application: 03737323.0
(71) Applicant: Novartis AG, 4056 Basel (CH); Novartis Pharma GmbH, 1230 Wien (AT)
(72) Inventor: Fozard, John, R., 68220, Hegenheim (FR); Weber, Eckhard, 4054, Basel (CH); Pfannkuche, Hans-Jürgen, 79576, Weil (DE)
(74) Representative: Strang, Andrea Josephine

(57) **Abstract**

The use of a compound of formula II 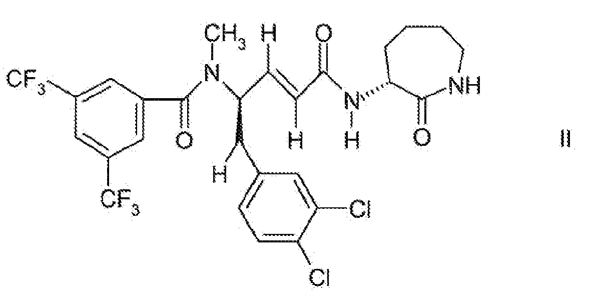 in free form or in the form of a pharmaceutically acceptable salt for the preparation of a medicament for the treatment of a functional motility disorder of the viscera.

## Description

This invention relates to organic compounds and, in particular to their use as pharmaceuticals.

The invention provides, in one aspect, use of a compound of formula I 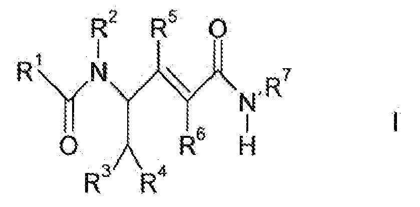 in free form or in the form of a pharmaceutically acceptable salt for the preparation of a medicament for the treatment of a functional motility disorder of the viscera, wherein
R¹ is phenyl that is unsubstituted or is substituted by 1, 2 or 3 substituents selected from the group halogen, C₁-C₇-alkyl, trifluoromethyl, hydroxy and C₁-C₇-alkoxy,
R² is hydrogen or C₁-C₇-alkyl,
R³ is hydrogen, C₁₋C₇-alkyl or phenyl that is unsubstituted or is substituted by 1, 2 or 3 substituents selected from the group halogen, C₁-C₇-alkyl, trifluoromethyl, hydroxy and C₁-C₇-alkoxy,
R⁴ is phenyl that is unsubstituted or is substituted by 1, 2 or 3 substituents selected from the group halogen, C₁-C₇-alkyl, trifluoromethyl, hydroxy and C₁-C₇-alkoxy; or is naphthyl, 1H-indol-3-yl or 1-C₁-C₇-alkyl-mdol-3-yl,
R⁵ and R⁶ are each independently of the other hydrogen or C₁-C₇-alkyl, at least one of R⁵ and R⁶ being hydrogen, and
R⁷ is C₃-C₈-cycloalkyl, D-azacycloheptan-2-on-3-yl or L-azacycloheptan-2-on-3-yl.

The invention provides, in another aspect, a method of treating a functional motility disorder of the viscera, in a subject, particularly a human subject, in need of such treatment, which comprises administering to said subject an effective amount of a compound of formula I as hereinbefore defined.

Treatment of a functional motility disorder of the viscera in accordance with the invention may be symptomatic or prophylactic (preventative).

Functional motility disorders of the viscera to be treated in accordance with the invention include those associated with visceral hypersensitivity and/or altered motor responses (including electrolyte/water secretion), for example functional bowel disorders and functional gastrointestinal disorders, such as irritable bowel syndrome (IBS), constipation, diarrhoea, functional dyspepsia, gastro-oesophageal reflux disease, functional abdominal bloating, and functional abdominal pain, other conditions associated with visceral hypersensitivity such as post-operative visceral pain, visceral smooth muscle spasms, and irritable bladder and other functional bowel disorders (not necessarily associated with visceral hypersensitivity or abnormal motor responses). The invention is of particular importance for the treatment of irritable bowel syndrome (IBS), especially diarrhoea-predominant IBS, and functional dyspepsia (FD).

The general terms used hereinabove and hereinbelow preferably have the following meanings:
C₁-C₇-alkyl is, for example, methyl, ethyl, n-propyl, isopropyl, n-butyl, isobutyl, sec-butyl, tert-butyl, n-pentyl, neopentyl, n-hexyl or n-heptyl, preferably C₁-C₄ alkyl, especially methyl or ethyl, and more especially methyl.
Halogen is, for example, fluorine, chlorine, bromine or iodine.
Halophenyl is, for example, (fluoro-, chloro-, bromo- or iodo-)phenyl, preferably fluorophenyl or chlorophenyl, especially 4-fluorophenyl or 4-chlorophenyl, and more especially 4-chlorophenyl.
Dihalopbenyl is, for example, dichlorophenyl, difluorophenyl or chlorofluorophenyl, preferably dichlorophenyl or difluorophenyl, especially 3,4-dichlorophenyl or 3,4-difluorophenyl, and more especially 3,4-dichlorophenyl.
Trihalophenyl is, for example, trifluorophenyl or trichlorophenyl.
1-C₁-C₇-alkyl-indol-3-yl is, for example, 1-methyl-indol-3-yl.
C₃-C₈-Cycloalkyl- and analogously G₅-C₇-cycloalkyl- is in each case a cycloalkyl radical having the number of ring carbon atoms indicated. C₃-C₈-Cycloalkyl is therefore, for example, cyclopropyl, cyclobutyl, cyclopentyl, cyclohexyl, cycloheptyl or cyclooctyl, preferably cyclohexyl.
D-Azacyclobeptan-2-on-3-yl corresponds to the following group 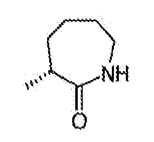 which is derived from D(+)-epsilon-caprolactam (amino-)substituted in the 3-position [≈ D-3-amin-epsilon-caprolactam = (R)-3-amino-hexahydro-2-azepinone].Analogously, L-aza-cycloheptan-2-on-3-yl corresponds to the group 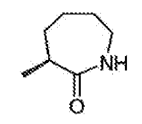 which is derived from L(-)-epsilon-caprolactam (amino-)substituted in the 3-position [≈ L-3-amino-epsilon-caprolactam = (S)-3-amino-hexahydro-2-azepinone].

The compounds of formula I may be of formula IA 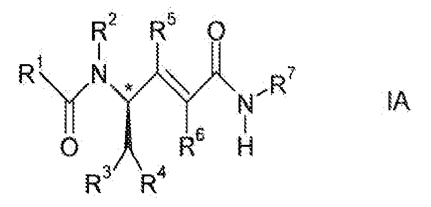 where * denotes the R configuration, or of formula IB 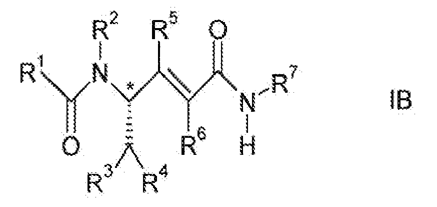 where * denotes the S configuration, where R¹, R², R³, R⁴, R⁵, R⁶ and R⁷ are as hereinbefore defined.

Compounds of formula IA are usually preferred for use in accordance with the invention Compounds of formula I having a basic group may, for example, form acid addition salts with suitable mineral acids, such as hydrohalic acids, sulfuric acid or phosphoric acid, for example hydrochlorides, hydrobromides, sulfates, hydrogen sulfates or phosphates. Where the compounds of formula I contain an acid group, corresponding salts with bases are also possible, for example corresponding alkali metal or alkaline earth metal salts, for example sodium, potassium or magnesium salts, or salts with ammonia or organic amines, for example ammonium salts.

The invention relates preferably to the use of compounds of formula I wherein
R¹ is phenyl, 3,5-bistrifluoromethyl-phenyl or 3,4,5-trimethoxyphenyl,
R² is hydrogen or C₁-C₇-alkyl,
R³ is hydrogen or phenyl,
R⁴ is phenyl, halo-phenyl, dihalo-phenyl, trihalo-phenyl, 2-naphthyl, 1H-indol-3-yl or 1-C₁-C₇-alkyl-indol-3-yl,
R⁵ and R⁶ are each independently of the other hydrogen or C₁-C₇-alkyl, at least one of R⁵ and R⁶ being hydrogen, and
R⁷ is C₅-C_{7c}ycloalkyl, D-azacycloheptan-2-on-3-yl or L-azacyclohcptan-2-on-3-yl.

The invention relates especially to the use of compounds of formula I wherein
R¹ is 3,5-bistrifluoromethyl-phenyl,
R² is hydrogen, methyl or ethyl,
R³ is hydrogen or phenyl,
R⁴ is phenyl, 4-chlorophenyl, 4-fluorophenyl, 3,4-dichloro-phenyl, 3,4-difluoro-phenyl, 3-fluoro-4-chloro-phenytl 3,4,5,-trifluoro-phenyl, 2-naphthyl, 1H-indol-3-yl or 1-methyl-indol-3-yl,
R⁵ and R⁶ are each independently of the other hydrogen or methyl, at least one of R⁵ and R⁶ being hydrogen, and
R⁷ is cyclohexyl, D-azacycloheptan-2-on-3-yl or L-azacycloheptan-2-on-3-yl.

The invention relates more especially to the use of compounds of formula I Wherein
R¹ is 3,5-bistrifluoromethyl-phenyl,
R² is hydrogen or methyl,
R³ is hydrogen or phenyl,
R⁴ is phenyl, 4-chlorophenyl, 3,4-dichloro-phenyl, 2-naphthyl, 1H-indol-3-yl or 1-methyl-indol-3-yl,
R⁵ and R⁶ are hydrogen, and
R⁷ is cyclohexyl, D-azacycloheptan-2-on-3-yl or L-azacycloheptan-2-on-3-yl.

Special mention should be made of each of the following sub-groups of a group of compounds of formula I:
(1) compounds of formula I wherein R⁷ is D-azacycloheptan-2-on-3-yl; (2) compounds of formula I wherein R⁵ and R⁶ are hydrogen; (3) compounds of formula I wherein R¹ is phenyl, 3,5-bistrifluoromethyl-phenyl or 3,4,5-trimethoxyphenyl; (4) compounds of formula I in free form, that is to say not in the form of a salt.

Specific examples of compounds of formula I include
(4R)[N'-methyl-N'-(3,5-bistrifluoromethyl-benzoyl)-ammo]-5-(1-methyl-indol-3-yl)-pent-2-enoic acid N-[(R)-epsilon-caprolactam-3-yl]-amide,
(4R)-[N'-methyl-N'-(3,5-bistrifluoromethyl-benzoly)-amino]-5-(methyl-indol-3-yl)-pent-2-enoic acid N-[(S)-epsilon-caprolactam-3-yl]-amide,
(4R)-[N'-methyl-N'-(3,5-bistrifluoromethyl-benzoyl)-amino]-5-(1-methyl-indol-3-yl)-pent-2-enoic acid N-cyclohexyl-amide,
(4R)-[N'-methyl-N'(3,5-bistrifluoromethyl-benzoyl)-amino]-5-(1-methyl-indol-3-yl)-2-methyl-pent-2-enoic acid N-cyclohexyl-amide,
(4R)-[N'-methyl-N'-(3,5-bistrifluoromethyl-benzoly)-amino]-5-(1-methyl-indol-3-yl)-2-methyl-pent-2-enoic acid N-[(R)-epsliton-caprolactam-3-yl]-amide,
(4R)-[N'-methyl-N'-(3,5-bistrifluoromethyl-benzoyl)-amino]-5-(1-methyl-indol-3-yl)-2-methyl-pent-2-enoic acid N-[(S)-epsilon-caprolactam-3-yl]- amide,
(4R)-(N'-methyl-N'-benzoyl-amino)-5-(1-methyl-indol-3-yl)-2-methyl-pent-2-enoicacid N-[(R)-epsilon-caprolactam-3-yl]-amide,
(4R)-[N'-methyl-N'-(3,5-bistrifluoromethyl-benzoyl)-amino]-5-(naphth-2-yl)-pent-2-enoic acid N-[(R)-epsilon-caprolactam-3-yl]-amide,
(4R)-(N'-methyl-N'-benzoyl)-amino-5-(naphth-2-yl)-pent-2-enoic acid N-[(R)-epsilon-caprolactam-3-yl]-amide,
(4R)-[N'-methyl-N'-(3,5-bistrifluoromethyl-benzoyl)-amino]-5-(naphth-2-yl)-2-methyl-pent-2-enoic acid N-[(R)-epsilon-caprolactam-3-yl]-amide,
(4R)-[N'-methyl-N'-(3,4,5-trimethoxy-benzoly)-amino]-5-(naphth-2-yl)-2-methyl-pent-2-enoic acid N-[(R)-epsilon-caprolactam-3-yl]-amide,
(4R)-[N'-methyl-N'-(3,5-bistrfluoromethyl-benzoyl)-amino]-5-(napth-2-yl)-2-methyl-pent-2-enoic acid N-[(s)-epsilon-caprolactam-3-yl]-amide,
(4R)-[N'-methyl-N'-(3,5-bistrifluoromethyl-benzoyl)-amino]-5-(naphth-2-yl)-2-methyl-pent-2-enoic acid N-cyclohexyl-amide,
(4R)-[N'-methyl-N'-(3,5-bistrifluoromethyl-benzoyl)-amino]-5-(1-methyl-indol-3-yl)-pent-2-enoic acid N-[(S)-epsilon-caprolactam-3-yl]-amide,
(4R)-[N'-methyl-N'-(3,5-bistrifluoromethyl-benzoyl)-amino]-4-(4-chlorobenzyl)-but-2-enoic acid N-[(R)-epsilon-caprolactan-3-yl]-amide,
(4R)-[N'-methyl-N'(3,5-bistrifluoromethyl-benzoyl)-amino]-4-(4-chlorobenzyl)-but-2-enoic acid N-[(S)-epsilon-caprolactam-3-yl]-amide,
(4R)-[N'-methyl-N'-(3,5-bistrifluoromethyl-benzoyl)-amino]-4-(4-chlorobenzyl)-but-2-enoic acid N-cyclohexyl-amide,
(4R)-[N'-methyl-N'-(3,5-bistrifluoromethyl-benzoyl)-amino]-4-(3,4-dichlorobenzyl)-but-2-enoic acid N-cyclohexyl-amxie,
(4R)-[N'-methyl-N'-(3,5-bistrifluoromethyl-benzoyl)-amino]-4-(3,4-difluorobenzyl)-but-2-enoic acid N-cyclohexyl-amide,
(4R)-[N'-methyl-N'-(3,5-bistriflroromethyl-benzoly)-amino]-5-(4-chlorophenyl)-2-methylpent-2-cnoic acid N-cyclohexylamide,
(4R)-[N'-methyl-N'-(3,5-bistriflroromethyl-benzoly)-amino]-5-(4-chlorophenyl)-2-methylpent-2-enoic acid N-[(R)-epsilon-caprolactam-3-yl]-amide,
(4R)-[N'-methyl-N'-(3,5-bistrifluoromethyl-benzoly)-amino]-4-(4-chlorophenyl)-2-methylbut-2-enoic acid [(S)-epsilon-caprolactam-3-yl]-amide,
(4R)-[N'-ethyl-N'-(3,5-bistriflroromethyl-benzoly)-amino]-5-(4-chlorophenyl)-2-enoic acid N-[(S)-epsilon-caprolactam-3-yl]-amide,
(4R)-[N'-methyl-N'-(3,5-bistriflroromethyl-benzoly)-amino]-5-(4-chlorophenyl)-3-methylpent-2-enoic acid N-cyclohexyl-amide,
(4R)-[N'-methyl-N'-(3,5-bistriflroromethyl-benzoly)-amino]-4-(4-chlorophenyl)-3-methylbut-2-enoic acid [(R)-epsilon-caprolactam-3-yl]-amide,
(4R)-[N'-methyl-N'-(3,5-bistriflroromethyl-benzoly)-amino]-4-(4-chlorophenyl)-but-2-enoic acid N-[(R)-epsilon-caprolactam-3-yl]-amide,
(4R)-[N'-methyl-N'-(3,5-bistriflroromethyl-benzoly)-amino]-4-(3,4-chlorophenyl)-but-2-enoic acid N-[(R)-epsilon-caprolactam-3-yl]-amide,
(4R)- and (4S)-4-[N'-methyl-N'-(3,5-bistriflroromethyl-benzoly)-amino]-4-(3-fluoro-4-chlorobenzyl)-but-2-enoic acid N-[(R)-epsilon-caprolactam-3-yl]-amide,
(4R)- and (4S)-4-[N'-methyl-N'-(3,5-bistriflroromethyl-benzoly)-amino]-4-(3,4-difluorobenzyl)-but-2-enoic acid N-[(R)-epsilon-caprolactam-3-yl]-amide,
(4R)- and (4S)-[N'-methyl-N'-(3,5-bistriflroromethyl-benzoly)-amino]-4-(3,4-dibromobenzyl)-but-2-enoic acid N-[(R)-epsilon-caprolactam-3-yl]-amide,
(4R)- and (4S)-[N'-methyl-N'-(3,5-bistriflroromethyl-benzoly)-amino]-4-(3,4,5-trifluorobenzyl)-but-2-enoic acid N-[(R)-epsilon-caprolactam-3-yl]-amide,
(4R)- and (4S)-[N'-methyl-N'-(3,5-bistriflroromethyl-benzoly)-amino]-4-(4-fluorobenzyl)-but-2-enoic acid N-[(R)-epsilon-caprolactam-3-yl]-amide,
(4R)- and (4S)-[N'-(3,5-bistriflroromethyl-benzoly)-N'-methyl-amino]-5,5-diphenyl-pent-2-enoic acid N-[(S)-epsilon-caprolactam-3-yl]-amide,
(4S)-4-[N'-methyl-N'-(3,5-bistriflroromethylbenzoly)-amino]-4-(3,4-dichlorobenzyl)-but-2-enoic acid N-[(R)-epsilon-caprolactam-3-yl]-amide,
(4R)-4-[N'-methyl-N'-(3,5-bistriflroromethylbenzoly)-amino]-4-(3,4-dichlorobenzyl)-but-2-enoic acid N-[(S)-epsilon-caprolactam-3-yl]-amide, and
(4S)-4-[N'-methyl-N'-(3,5-bistriflroromethylbenzoly)-amino]-4-(3,4-dichlorobenzyl)-but-2-enoic acid N-[(S)-epsilon-caprolactam-3-yl]-amide.

The invention relates most importantly to the use of (4R)-4-[N'-methyl-N'-(3,5-bistriflroromethylbenzolylbenzoly)-amino]-4-(3,4-dichlorobenzyl)-but-2-enoic acid N-[(R)-epsilon-caprolactam-3-yl]-amide, i.e. a compound of formula

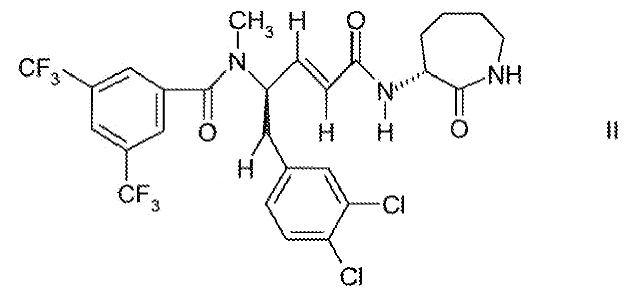

The compounds of formula I, in free or pharmaceutically acceptable salt form, may be prepared as described in WO 98/07694 or WO 01/85696. As mentioned therein, they may be in the form of their hydrates and/or may include other solvents, for example solvents which may have been used for the crystallisation of compounds in solid form.

Depending upon the nature of the variables and the corresponding number of centres of asymmetry and also upon the staring materials and procedures chosen, the compounds of formula I may be obtained in the form of mixtures of stereoisomers, for example mixtures of diastereoisomers or mixtures of enantiomers, such as racemates, or possibly also in the form of pure stereoisomers. Mixtures of diastereoisomers obtainable in accordance with the process or by some other method can be separated in customary manner into mixtures of enantiomers, for example racemates, or into individual diastereoisomers, for example on the basis of the physico-chemecal differences between the constituents in known manner by fractional crystallisation, distillation and/or chromatography. Advantageously the more active isomer is isolated.

Mixtures of enantiomers, especially racemates, obtainable in accordance with the process or by some other method can be separated into the individual enantiomers by known methods, for example by recrystallisation from an optically active solvent, with the aid of microorganisms, by chromatography and/or by reaction with an optically active auxiliary compound, for example a base, acid or alcohol, to form mixtures of diastereoisomeric salts or functional derivatives, such as esters, separation thereof and freeing of the desired enantiomer. Advantageously the more active enantiomer is isolated.

In the treatment of disorders in accordance with the invention, compounds of formula I, in free form or in pharmaceutically acceptable salt form, may be administered by any appropriate route, for example orally, e.g. in tablet, capsule or liquid form, parenterally, for example in the form of an injectable solution or suspension, or intranasally, for example in the form of an aerosol or other atomisable formulation using an appropriate intranasal delivery device, e.g. a nasal spray such as those known in the art.

The compound of formula I in free or salt form may be administered in a pharmaceutical composition together with a pharmaceutically acceptable diluent or carrier. Such compositions may be as described in WO 98/07694, for example tablets, capsules, liquids, injection solutions, infusion solutions or inhalation suspensions as described in Examples A to E of WO 98/07694, or may be prepared using other formulating ingredients and techniques known in the art.

The dosage of the compound of formula 1 in free or salt form can depend on various factors, such as the activity and duration of action of the active ingredient, the severity of the condition to be treated, the mode of administration, the species, sex, ethnic origin, age and weight of the subject and/or its individual condition. In a normal case the daily dose for administration, for example oral administration, to a warm-blooded animal, particularly a human being, weighing about 75 kg is estimated to be from approximately 1 mg to approximately 1000 mg, especially from approximately 5 mg to approximately 200 mg. That dose may be administered, for example, in a single dose or in several part doses of, for example, from 5 to 100 mg,
The utility of a compound of formula I in the treatment of the disorders hereinbefore described may be demonstrated in an in vivo model of visceral hypersensitivity, for example as described hereinafter in Example 1, in a peristaltic reflex model, for example as described hereinafter in Example 2, or an epithelial secretion model, for example as described hereinafter in Example 3.

The invention is illusrated by the following Examples.

### Example 1

In conscious guinea pigs, two experimental paradigms are applied to induce visceral hypersensitivity, *i)* restraint stress (immobilization of the animals in a tube) and *ii)* colonic tissue irritation (colonic instillation of acetic acid, 0.6 % in saline, 1.5 ml, 2 cm proximal to the anus). Colorectal distension is performed by inflating a balloon to a net pressure (at the colonic wall) ot 20 mmHg (26.7 mbar) for 10 minutes before and following induction of visceral hypersensitivity. During the distension period the visceromotor response, i.e. the number and quality of abdominal contractions (body arching and lifting of pelvic structures) are recorded and quantified in a blinded fashion (Al-Chaer et al., Gastroenterology 2000; 119: 1276-1285). Following the performance of baseline colorectal distension protocols, vehicle or the compound of formula II (3 and 10 mg/kg) are dosed orally one hour prior to the second colorectal distensions, which are done after the induction of visceral hypersensitivity. The effects of vehicle and the compound of formula II are assessed in 6-8 animals. Restraint stress and local tissue irritation by intracolonic instillation of acetic acid significantly exaggerates the visceromotor responses upon colorectal distension by 54.5 % and 29.1 %, respectively, over baseline. The compound of formula II reverses the exaggerated behavioural pain responses upon colorectal balloon distensions in both restraint stress-induced and tissue irritation-induced colonic hypersensitivity. The reversal in pain responses is statistically significant at both doses rested, 3 and 10 mg/kg p.o. (p<0.05; ANOVA post-hoc Dunnett's test).

### Example 2

Substance P - mediated exaggerated peristalsis is studied in Mayflower organ baths using isolated segments of guinea pig ileum (Holzer et al., J. Pharmacol. Exp. Ther. 1995; 274: 322-328), The lumen of ileal segments is perfused with Krebs-Henscleit solution, and intraluminal pressures are continuously recorded. During perfusion, each ileal segment fills gradually, and hence, the intraluminal pressures rise until they reach a threshold at which peristalsis is triggered, i.e. an aborally moving wave of peristaltic contractions. Any wave of peristaltic contractions results in a spike-like increase in intraluminal pressure and causes a partial emptying of fluid from the segment. Pressure thresholds triggering peristaltic contractions are used to quantify the effects of the compound of formula II. Cumulative application of substance P (1 nM up to 30 µM evokes exaggerated peristaltic events by lowering the thresholds necessary to trigger peristaltic contractions. The effects of substance P are concentration-dependent with a pD₂ value of 7.20. The compound of formula II (30 nM and 100 nM) competitively inhibits the substance P - evoked exaggerated peristalsis with apparent pA₂ values of 7.35 and 7.23 respectively.

### Example 3

Epithelial secretion is tested in submucosa/mucosa preparations of guinea pig colon. Using Ussing chamber techniques (Frieling et al., Naunyn Schmiedebergs Arch. Pharmacol. 1999; 359: 71-79) short circuit currents are recorded, and epithelial secretion (electrogenic chloride secretion) is stimulated via cumulative treatment with substance P (0.1 nM up to 10 µM); it triggers secretion (increases in short circuit currents) in a concentration-dependent fashion (pD₂ = 7.50 ). The compound of formula II (30 nM, 100 nM, and 300 nM) competitively inhibits substance P - induced electrogenic chloride secretion; a Schild plot analysis reveals a pA₂ value of 7.94.

### Further Embodiments of the Invention

1. The use of a compound of formula I 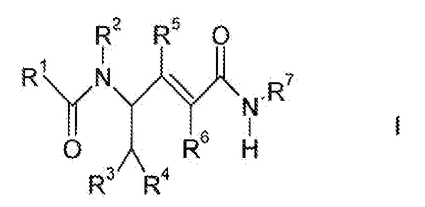 in free form or in the form of a pharmaceutically acceptable salt for the preparation of a medicament for the treatment of a functional motility disorder of the viscera, wherein
   R¹ is phenyl that is unsubstituted or is substituted by 1, 2 or 3 substituents selected from the group halogen, C₁-C₇-alkyl, trifluoromethyl, hydroxy and C₁-C₇-alkoxy,
   R² is hydrogen or C₁-C₇-alkyl,
   R³ is hydrogen, C₁-C₇alkyl or phenyl that is unsubstituted or is substituted by 1, 2 or 3 substituents selected from the group halogen, C₁-C₇-alkyl, trifluoromethyl, hydroxy ant C₁-C₇-alkoxy,
   R⁴ is phenyl that is unsubstituted or is substituted by 1, 2 or 3 substituents selected from the group halogen, C₁-C₇-alkyl, trifluoromethyl, hydroxy and C₁-C₇-alkoxy; or is naphthyl, 1H-indol-3-yl or 1-C₁-C₇-alkyl-indol-3-yl,
   R⁵ and R⁶ are each independently of the other hydrogen or C₁-C₇-alkyl, at least one of R⁵ and R⁶ being hydrogen, and
   R⁷ is C₃-C₈-cycloalkyl, D-azacycloheptan-2-on-3-yl or L-axscycloheptan-2-on-3-yl.
2. Use according to embodiment 1, in which the compound of formula I is of formula 1A 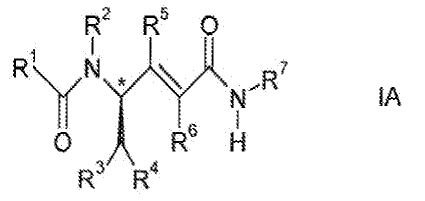 where * denotes the R configuration and R¹, R², R³, R⁴, R⁵, R⁶ and R⁷ are as defined in claim 1.
3. Use according to embodiment 1 or 2, in which
   R¹ is phenyl, 3,5-bistrifluoromethyl-phenyl or 3,4,5-trimethoxyphenyl,
   R² is hydrogen or C₁-C₇-alkyl,
   R³ is hydrogen or phenyl,
   R⁴ is phenyl, halo-phenyl, dihalo-phenyl, trihalo-phenyl, 2-naphthyl, 1H-indol-3-yl or 1-C₁-C₇-alkyl-indol-3-yl,
   R⁵ and R⁶ are each independently of the other hydrogen or C₁-C₇-alkyl, at least one of R⁵ and R⁶ being hydrogen, and
   R⁷ is C₅-C₇cycloalkyl, D-azacycloheptan-2-on-3-yl or L-azacycloheptan-2-on-3-yl.
4. Use according to embodiment 1 or 2, in which
   R¹ is 3,5-bistrifluoromethyl-phenyl,
   R² is hydrogen, methyl or ethyl,
   R³ is hydrogen or phenyl,
   R⁴ is phenyl, 4-chlorophenyl, 4-fhorophenyl, 3,4-dichloro-phenyl, 3,4-diflnoro-phenyl, 3-fluoro-4-chloro-phenyl, 3,4,5-trifluoro-phenyl, 1-naphthyl, 1H-indol-3-yl or 1-methyl-indol-3-yl,
   R⁵ and R⁶ are each independently of the other hydrogen or methyl, at least one of R⁵ and R⁶ being hydrogen, and
   R⁷ is cyclohexyl, D-azacycloheptan-2-on-3-yl or L-azacycloheptan-2-on-3-yl,
5. Use according to embodiment 1 or 2, in which
   R¹ is 3,5-bistrifluoromethyl-phenyl,
   R² is hydrogen or methyl,
   R³ is hydrogen or phenyl,
   R⁴ is phenyl, 4-chlorophenyl, 3,4-dichloro-phenyl, 2-naphthyl, 1H-indol-3-yl or 1-methyl-indol-3-yl,
   R⁵ and R⁶ are hydrogen, and
   R⁷ is cyclohexyl, D-azacycloheptan-2-on-3-yl or L-azacyclohepran-2-on-3-yl.
6. Use according to em bodiment 1, in which the compound of formula I is a compound of formula 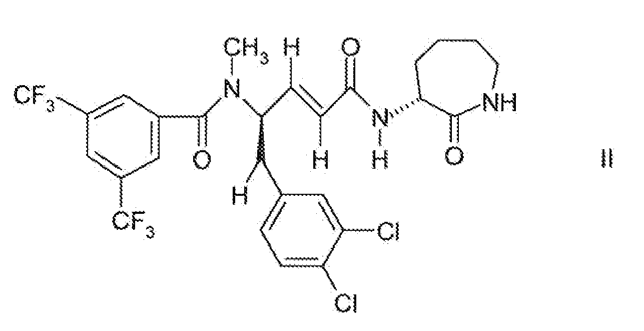
7. Use according to any one of embodiments 1 to 6, in which the disorder is associated with visceral hypersensitivity and/or altered motor responses.
8. Use according to embodiment 7, in which the disorder is a functional bowel disorder or functional gastrointestinal disorder.
9. Use according to embodiment 8, in which the disorder is irritable bowel syndrome or functional dyspepsia.

## Claims

1. The use of a compound of formula II: 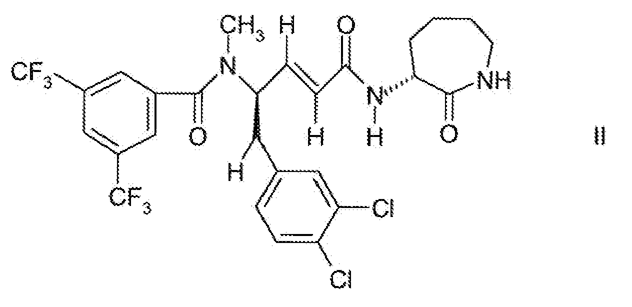 in free form or in the form of a pharmaceutically acceptable salt, for the preparation of a medicament for the treatment of a functional motility disorder of the viscera.

2. Use according to claim 1, in which the disorder is-associated with visceral hypersensitivity and/or altered motor responses.

3. Use according to claim 2, in which the disorder is a functional bowel disorder or functional gastrointestinal disorder.

4. Use according to claim 3, in which the disorder is irritable bowel syndrome or functional dyspepsia.

5. Use according to claim 4, in which the disorder is irritable bowel syndrome.
